# EUROPÄISCHE PATENTSCHRIFT

(11) **EP 2 736 569 B1**
(45) Veröffentlichungstag und Bekanntmachung des Hinweises auf die Patenterteilung: **03.06.2015**
(21) Anmeldenummer: 12727673.1
(22) Anmeldetag: 15.06.2012
(51) Int. Cl.: A61M 15/00, H02K 7/116, H02K 7/18, H02K 35/02, F16H 21/44, B05B 12/00, B05B 11/00, G02F 1/133

(54) **Austragsvorrichtung für Medien**
Discharge device for media
Dispositif de distribution de fluides

(30) Priorität: 27.07.2011 DE 102011079949
(43) Veröffentlichungstag der Anmeldung: 04.06.2014
(73) Patentinhaber: Aptar Radolfzell GmbH, 78315 Radolfzell (DE)
(72) Erfinder: KOHNLE, Jörg, 78056 Villingen-Schwenningen (DE); KÖRNER, Joachim, 88690 Uhldingen-Mühlhofen (DE)
(74) Vertreter: Patentanwälte Ruff, Wilhelm, Beier, Dauster & Partner
(86) Internationale Anmeldenummer: PCT/EP2012/061519
(87) Internationale Veröffentlichungsnummer: WO 2013/013890

(56) Entgegenhaltungen:
- EP-B1- 1 559 083
- WO-A1-2007/137991
- US-A1- 2007 017 506
- US-A1- 2007 135 756

## Beschreibung

Die Erfindung betrifft eine Austragvorrichtung für flüssige, pastöse oder pulverförmige Medien mit einem Gehäuse mit einem ersten Gehäuseabschnitt und einem zweiten Gehäuseabschnitt, die gegeneinander zum Zwecke der Betätigung hubbeweglich sind, mit einer Austragsöffnung zum Austrag des Mediums und mit einem Reservoir zur Speicherung des Mediums vor dessen Austrag. Dabei ist bei einer gattungsgemäßen Austragvorrichtung vorgesehen, dass durch eine manuelle Hubbewegung der Gehäuseabschnitte gegeneinander eine Förderung von Medium vom Reservoir zur Austragöffnung bewirkt werden kann. Weiterhin ist vorgesehen, dass die Austragvorrichtung einen elektrischen Verbraucher und einen elektromagnetischen Generator zur Umwandlung einer Teils der bei der Betätigung eingebrachten mechanischen Energie in elektrische Energie zur Versorgung des elektrischen Verbrauchers aufweist. Dabei weist ein solcher elektromagnetischer Generator in der Art eines Dynamos eine Komponente auf, die einen Magneten umfasst, und zweite Komponente, die einen mit dem elektrischen Verbraucher verbundenen Leiter, insbesondere in Form einer Spule, umfasst, wobei eine der Komponenten ortsfest zu einem der Gehäuseabschnitte und eine der Komponenten gegenüber der ortsfesten Komponente beweglich vorgesehen ist, so dass durch eine Relativbewegung der Komponenten eine Stromerzeugung möglich ist.

Gattungsgemäße Austragvorrichtungen können zum Austragen von verschiedenen Medien Verwendung finden, wobei es sich vorzugsweise um eine Austragvorrichtung handelt, welche mit einem pharmazeutischen Medium befüllt und zum Austrag dieses Mediums vorgesehen ist. Solche gattungsgemäßen Austragvorrichtungen finden in der Regel durch den Patienten oder den Nutzer selbst Verwendung. Es handelt sich um tragbare Einheiten, welche unproblematisch an jeden Ort mitgenommen werden können.

Der Austragvorgang wird bei gattungsgemäßen Austragvorrichtungen durch die Hubbeweglichkeiten zweier Gehäuseabschnitte ermöglicht. Diese Hubbewegung der Gehäuseabschnitte verursacht den Austrag, wobei dies beispielsweise dadurch realisiert sein kann, dass eine Pumpe, wie insbesondere beispielsweise eine Kolbenpumpe, durch die Hubbewegung betätigt wird. Alternative Gestaltungen können jedoch auch vorsehen, dass durch die Hubbewegung lediglich ein Austragventil geöffnet wird, durch welches dann bereits druckbeaufschlagt in der Austragvorrichtung gespeichertes Medium zur Austragöffnung hindurchströmen und in die umgebende Atmosphäre gelangen kann.

Bei gattungsgemäßen Austragvorrichtungen ist vorgesehen, dass diese einen elektrischen Verbraucher aufweisen. Derartige Austragvorrichtungen mit elektrischem Verbraucher werden zunehmend üblicher. Die elektrischen Verbraucher können dabei beispielsweise ein elektronisches Zählwerk und Anzeigemittel wie ein LC-Display, LEDs oder dergleichen umfassen. Auch ein Sperrmechanismus, der nach einem Austragvorgang einen weiteren Austragvorgang für einen gewissen Zeitraum verhindert, umfasst üblicherweise mindestens einen elektrischen Verbraucher. Auch Funk-Sendeeinrichtungen und Sensoreinrichtungen sind als elektrischer Verbraucher möglich, um die korrekte Verwendung des Spenders zu überwachen.

Bei gattungsgemäßen Spendern mit einem solchen elektrischen Verbraucher ist vorgesehen, dass diese zur Stromversorgung des elektrischen Verbrauchers eine Umwandlungseinrichtung zur Umwandlung der durch die Hubbewegung manuell eingebrachten mechanischen Energie in elektrische Energie aufweisen. Für solche Umwandiungseinrichtungen existieren im Stand der Technik verschiedene Vorschläge. So schlägt die DE 60312026 T2 bereits vor, in ähnlicher Art und Weise wie bei einem üblichen Feuerzeug eine piezoelektrische Umwandlungsvorrichtung zu verwenden. Aus der WO 2007/137991 A1 ist als Alternative hierzu bekannt, entsprechend der gattungsgemäßen Gestaltung einen elektromagnetischen Generator zu verwenden. Als elektromagnetischer Generator wird in diesem Kontext eine Teilkomponente verstanden, die durch die Relativbewegung eines Permanentmagneten gegenüber einem stromführenden Leiter oder einer Spule in diesem bzw. dieser eine Spannung induziert.

Die WO 2007/137991 A1 schlägt einen rotativ arbeitenden Generator vor, wobei die Koppelung dieses Generators an die Relativbewegung der dort vorgesehenen Betätigungshandhabe durch eine an der Betätigungshandhabe ortsfest angebrachte Zahnstange erfolgt, welche mit einem Zahnrad des Generators kämmt. Problematisch an dieser bekannten Gestaltung ist, dass bei gattungsgemäßen Austragvorrichtungen verwendbare besonders kleine Generatoren einen vergleichsweise hohen Wirkungsgrad nur dann erzielen, wenn sie mit hohen Geschwindigkeiten betrieben werden. Die aus der WO 2007/137991 A1 bekannte Lösung kann diese hohen Geschwindigkeiten systematisch nicht gewährleisten, da nicht ausgeschlossen werden kann, dass ein Benutzer die Betätigungshandhabe sehr langsam hinabdrückt. Zwar würden auch dann die beiden Komponenten des Generators gegeneinander bewegt. Aufgrund der geringen Geschwindigkeit würde jedoch nur wenig elektrische Energie erzeugt.

Aus der US 2007/0135756 A1 ist eine Injektionseinrichtung bekannt, bei der durch rotative Bewegung eines Magneten und einer Spule elektrische Energie erzeugt wird.

### Aufgabe und Lösung

Aufgabe der Erfindung ist es, eine gattungsgemäße Austragvorrichtung dahingehend weiterzubilden, dass diese eine größere Menge elektrischer Energie je Betätigung liefert und insbesondere in reproduzierbarer Art und Weise diese Energiemenge liefert.

Erfindungsgemäß ist hierfür vorgesehen, dass eine erfindungsgemäße Austragvorrichtung einen Federenergiespeicher mit einem gegenüber einem zweiten Gehäuseabschnitt beweglichen Spannglied und einer zwischen dem zweiten Gehäuseabschnitt und dem Spannglied wirkenden Feder aufweist. Dabei ist das Spannglied dafür ausgebildet, während der Hubbewegung mit dem ersten Gehäuseabschnitt gekoppelt zu sein, so dass ein Spannen der Feder während der Hubbewegung bewirkt wird. Weiterhin ist vorgesehen, dass im zumindest teilweise gespannten Zustand der Feder das Spannglied vom ersten Gehäuseabschnitt entkoppelbar ist, so dass ein Entspannen der Feder ermöglicht wird. Während dieses Entspannens der Feder ist erfindungsgemäß das Spannglied zumindest phasenweise mit der beweglichen Komponente des Generators wirkgekoppelt, so dass die Bewegung des Spanngliedes eine Bewegung der beweglichen Komponente des Generators gegenüber der ortsfesten Komponente des Generators bewirkt.

Erfindungsgemäß ist somit vorgesehen, dass mit dem ersten Gehäuseabschnitt, dem zweiten Gehäuseabschnitt und dem Spannglied des Federenergiespeichers insgesamt drei Teileinrichtungen vorgesehen sind, die zum Zwecke des Antriebs der beweglichen Komponente des Generators zumindest phasenweise jeweils gegeneinander bewegt werden. Bei der Relativbewegung der Gehäuseabschnitte gegeneinander, welche auch den Austragvorgang des Mediums bewirkt, ist das Spannglied mit dem ersten Gehäuseabschnitt gekoppelt, so dass es mit diesem gemeinsam gegenüber dem zweiten Gehäuseabschnitt bewegt wird. Dabei wird die vorzugsweise als Druckfeder ausgebildete Feder des Federenergiespeichers gespannt, da diese nicht nur mit dem Spannglied, sondern auch mit dem zweiten Gehäuseabschnitt verbunden ist oder sich an diesem abstützt. Im zumindest teilweise gespannten Zustand der Feder und vorzugsweise in einer definierten Relativlage der beiden Gehäuseabschnitte zueinander wird dann, vorzugsweise automatisch weggesteuert, kraftgesteuert oder energiegesteuert, eine Entkoppelung des Spanngliedes vom ersten Gehäuseabschnitt bewirkt, so dass das Spannglied relativ zum zweiten Gehäuseabschnitt unter der Wirkung der gespeicherten Federenergie wieder seine Ausgangslage einnimmt bzw. zumindest in die entsprechende Richtung verlagert wird. Zumindest während einer Teilphase dieser Bewegung ist eine Wirkkopplung zur beweglichen Komponente des Generators vorgesehen, so dass diese gegenüber der vorzugsweise zum zweiten Gehäuseabschnitt ortsfesten Komponente bewegt wird und dadurch die Stromerzeugung verursacht.

Da die Relativbewegung des Spannglieds nach der Entkoppelung vom ersten Gehäuseabschnitt und relativ zum zweiten Gehäuseabschnitt zumindest weitgehend unabhängig davon erfolgt, ob die Bewegung der Gehäuseabschnitte gegeneinander zuvor schnell oder langsam durchgeführt wurde, wird eine weitgehend reproduzierbare Relativbewegung der Komponenten des Generators gegeneinander erzielt. Darüber hinaus kann durch eine entsprechende Wahl der Federkennwerte eine sehr schnelle Relativbewegung des Spannglieds gegenüber dem zweiten Gehäuseabschnitt nach der Entkopplung vom ersten Gehäuseabschnitt bewirkt werden. Die hohe Geschwindigkeit und die hohe Reproduzierbarkeit gestatten die Erzielung eines hohen Wirkungsgrades, so dass vergleichsweise viel Energie zur Versorgung der elektrischen Verbraucher zur Verfügung steht.

Diese Energie muss nicht unmittelbar den elektrischen Verbrauchern zugeführt werden. Es können Komponenten zur Spannungsanpassung, wie beispielsweise ein Gleichrichter, ein Spannungswandler oder auch Zwischenspeicher wie ein Pufferkondensator oder ein Puffer-Akkumulator Verwendung finden.
Das Spannglied ist bei einer erfindungsgemäßen Gestaltung in gleicher Bewegungsrichtung gegenüber den Gehäuseabschnitten beweglich, wie diese gegeneinander beweglich sind. Die einfachste Ausgestaltung der Wirkkoppelung zwischen dem Spannglied und der beweglichen Komponente des Generators sieht daher vor, dass diese bewegliche Komponente unmittelbar am Spannglied vorgesehen ist und somit auch linearbeweglich gegenüber dem ersten Gehäuseabschnitt ist. In einem solchen Falle wäre der Generator als Lineargenerator auszubilden. So könnte insbesondere der Permanentmagnet fest mit dem Spannglied verbunden sein und somit während der Bewegung des Spanngliedes gegenüber dem ersten Gehäuseabschnitt ebenfalls gegenüber einer am ersten Gehäuseabschnitt vorgesehenen Spule bewegt werden. Von Vorteil ist allerdings eine Gestaltung, bei der die bewegliche Komponente des elektromagnetischen Generators gegenüber der ortsfesten Komponente drehbeweglich gelagert ist. In diesem Fall bedarf es allerdings eines Getriebes, welches die Linearbewegung des Spanngliedes in eine rotative Bewegung der beweglichen Komponente des Generators überführt. Dieses Getriebe umfasst vorzugsweise eine linear erstreckte Verzahnung am Spannglied und ein insbesondere koaxial und vorzugsweise fest mit der beweglichen Komponente des Generators verbundenes Zahnrad.

Die Entkoppelung des ersten Gehäuseabschnitts vom Spannglied nach Spannen der Feder kann grundsätzlich auch manuell erfolgen. Bei einer solchen Gestaltung würde die Feder während der Hubbewegung der Gehäuseabschnitte gespannt und anschließend erst dann entspannt, wenn dies durch eine manuelle Betätigung an einer beispielsweise gesonderten Handhabe durch den Benutzer bewirkt wird. Bevorzugt ist jedoch eine Gestaltung, bei der das Spannglied automatisch vom ersten Gehäuseabschnitt entkoppelt wird, wobei vorzugsweise das Spannglied und der erste Gehäuseabschnitt derart aufeinander abgestimmt sind, dass das Spannglied in einer definierten Entkoppelungsstellung der Hubbewegung automatisch entkoppelt wird. Somit findet die Erzeugung elektrischer Energie stets unmittelbar im Zuge der Betätigung der Austragvorrichtung und in Reaktion auf die durch den Benutzer bewirkten Betätigungsbewegung statt. Dies ist zweckmäßig, da somit beispielsweise unmittelbar nach einem Austrag ein Zählregister aktualisiert werden kann und dessen Inhalt auf einem LC-Display ausgegeben werden kann.

Wie bereits erwähnt, sind höhere Rotations- oder Lineargeschwindigkeiten bei sehr klein gebauten Generatoren zumeist von Vorteil. Die Entkoppelungsstellung und die Feder einer erfindungsgemäßen Austragvorrichtung sind daher vorzugsweise derart aufeinander abgestimmt, dass während des Entspannens der Feder die bewegte Komponente des Generators im Falle eines Lineargenerators mindestens eine Geschwindigkeit von 800 mm/s, vorzugsweise von 1000 mm/s, insbesondere vorzugsweise von mindestens 1200 mm/s erreicht. Bei einem Rotationsgenerator ist von Vorteil, wenn eine Rotationsgeschwindigkeit von mindestens 30 U/sec erreicht wird. Demgegenüber noch von Vorteil sind Umdrehungsgeschwindigkeiten von mindestens 50 U/sec, insbesondere vorzugsweise von mindestens 80 U/sec. Die Beeinflussung der Geschwindigkeit erfolgt zum Einen durch das dem Generator vorgeschaltete Getriebe, zum Anderen über die Auslegung der Feder und die Relativstellung der Gehäuseabschnitte, in der es zur Entkopplung kommt.

Die Wirkkoppelung, die zwischen der Bewegung des Spanngliedes und der Bewegung der beweglichen Komponente des Generators erfindungsgemäß zumindest phasenweise besteht, kann auch als dauerhaft bestehende Wirkkopplung vorgesehen sein, so dass das Spannglied und die bewegliche Komponente des Generators sich stets gleichzeitig bewegen. Demgegenüber von Vorteil kann jedoch eine Gestaltung sein, bei der die Wirkkopplung derart ausgebildet ist, dass sie nach der Entkoppelung noch vor Erreichen des Stillstandes des Spanngliedes endet. Somit kommt bei einer solchen Gestaltung während des Entspannens der Feder das Spannglied außer Eingriff mit der beweglichen Komponente des Generators. Dies gestattet ein Nachlaufen der beweglichen Komponente des Generators. Der in der Endlage eintretende Stillstand des Spanngliedes bewirkt keinen sofortigen Stillstand der beweglichen Komponente des Generators. Somit wird ein geringerer Teil der mechanisch eingebrachten Energie in Wärmeenergie umgewandelt und ein höherer Anteil steht für die Erzeugung elektrischer Energie zur. Verfügung. Insbesondere ist eine derartige nur phasenweise bestehende Wirkkopplung des Spanngliedes zur beweglichen Komponente des Generators über eine Verzahnung möglich, wobei die beiden verzahnungstragenden Teile vor Erreichen des Stillstandes des Spanngliedes außer Eingriff voneinander gebracht werden.

Bei einer besonders bevorzugten Gestaltung sind die beiden Gehäuseabschnitte entlang einer Betätigungsrichtung gegeneinander beweglich, die mit einer durch eine Austragrichtung des Mediums definierten Haupterstreckungsrichtung der Austragvorrichtung einen Winkel zwischen 70° und 110° einschließt. Somit ist die Vorrichtung als sogenannte Side-Actuation-Vorrichtung ausgebildet. Dies hat sich im Hinblick auf den zur Verfügung stehenden Bauraum und dessen Ausnutzung als vorteilhaft herausgestellt. Auch sind solche Austragvorrichtungen ergonomischer zu handhaben.

Wie bereits dargestellt, weist vorzugsweise eine erfindungsgemäße Austragvorrichtung zur Förderung des Mediums zur Austragöffnung eine Förderpumpe oder aber zum Auslass des Mediums zur Austragvorrichtung ein Auslassventil auf. Diese Förderpumpe bzw. dieses Austragventil ist vorzugsweise derart ausgebildet ist, dass ein Austrag des Mediums im Zuge einer Hubbewegung erst jenseits der Entkoppelungsstellung erfolgt.

Bei einer Betätigung kommt es somit erst dann zum Austrag von Medium, wenn die Energieerzeugung bereits erfolgt ist. Dies ist insbesondere im Zusammenhang mit einer elektronischen Zählung der Austragvorgänge von Vorteil, da es gewährleistet, dass die zur Zählung erforderliche Energie zur Verfügung steht. Ein Austrag von Medium vor der Entkoppelungsstellung geht mit der Gefahr einher, dass eine unvollständige Betätigung zwar den Austrag des Medium verursacht, dessen Zählung jedoch nicht ermöglicht.

Bei anderen Gestaltungen, insbesondere bei Gestaltungen, bei denen die Energieerzeugung nicht dem primären Zweck einer Zählung dient, kann es von Vorteil sein, die Entkopplungsstellung innerhalb der letzten 10% des Hubweges vorzusehen, um so eine möglichst große Menge mechanischer Energie in der Feder zu speichern, bevor der Energieerzeugung beginnt.

### Kurzbeschreibung der Zeichnungen

Weitere Aspekte und Vorteile der Erfindung ergeben sich außer aus den Ansprüchen auch aus der nachfolgenden Beschreibung eines bevorzugten Ausführungsbeispiels der Erfindung, welches anhand der Figuren erläutert wird. Dabei zeigen:
- Fig. 1: eine erfindungsgemäße Austragvorrichtung in einer Explosionsdarstellung,
- Fig. 1a: die Austragvorrichtung der Fig. 1 in einer geschnittenen Darstellung mit teilweise ausgeblendetem innerem Gehäuseabschnitt,
- Fig. 2a bis 2c: die Austragvorrichtung der Fig. 1 vor Beginn einer Austragbetätigung,
- Fig. 3a bis 3c: die Austragvorrichtung kurz vor Auslösen eines internen Spannglieds zur Erzeugung elektrischer Energie,
- Fig. 4a bis 4c: die Austragvorrichtung während einer Hauptphase der Erzeugung elektrischer Energie und
- Fig. 5a bis 5c: die Austragvorrichtung während einer Nachlaufphase zur Erzeugung elektrischer Energie.

### Detaillierte Beschreibung des Ausführungsbeispiels

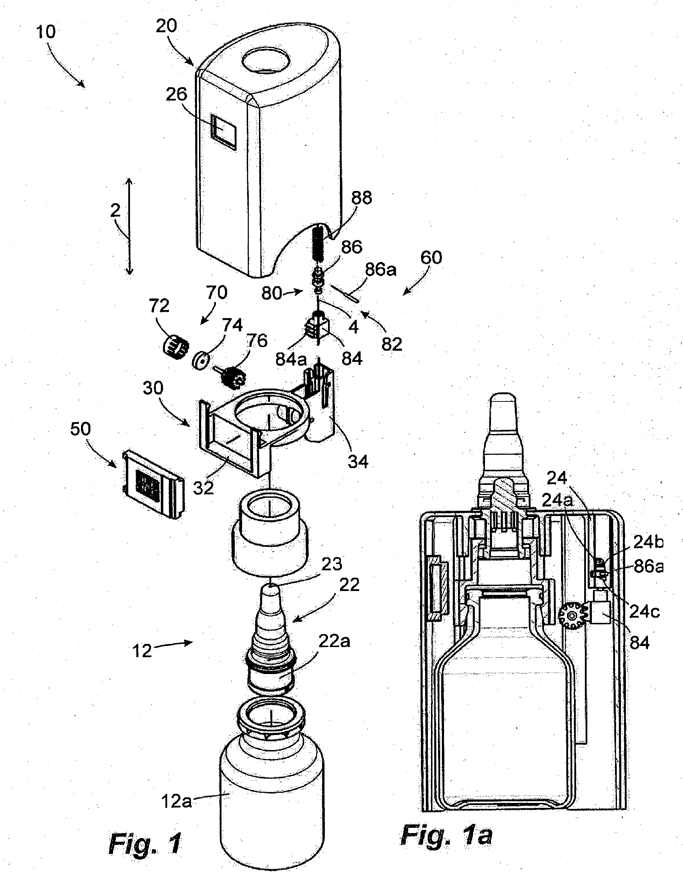

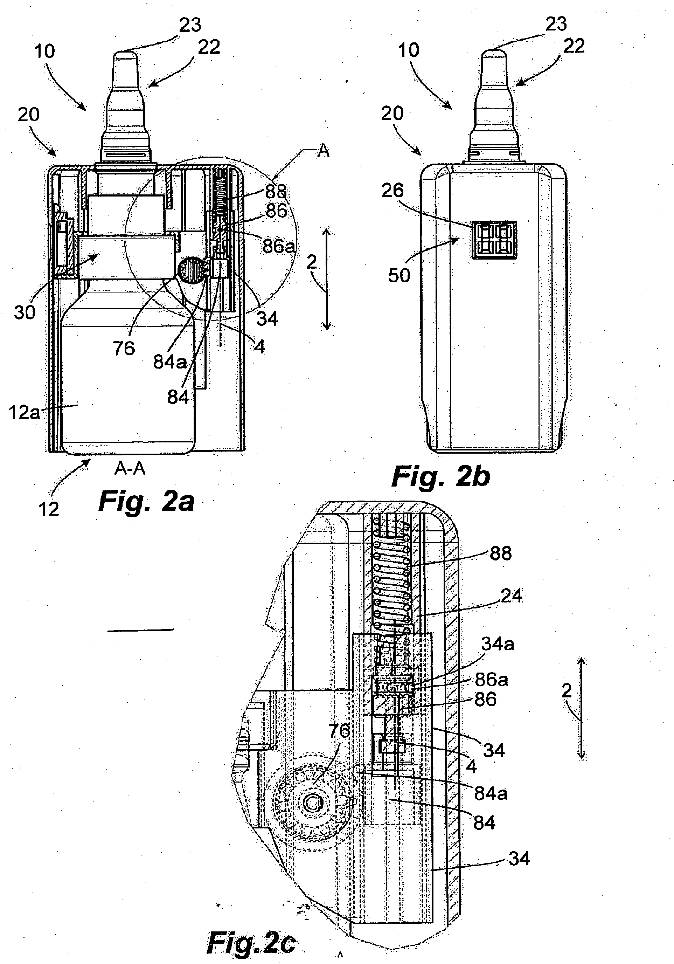

Fig. 1 zeigt eine Ausführungsform einer erfindungsgemäßen Austragvorrichtung in einer Explosionsdarstellung. Anhand dieser Fig. 1, der Fig. 1 a sowie der Fig. 2a werden zunächst die wesentlichen Komponenten dieser Austragvorrichtung 10 verdeutlicht.

Die Austragvorrichtung 10 verfügt über ein zweiteiliges Gehäuse mit einem inneren ersten Gehäuseabschnitt 30 und einem äußeren zweiten Gehäuseabschnitt 20. Der äußere Gehäuseabschnitt 20 bildet den überwiegenden Anteil der von außen sichtbaren Flächen der Austragvorrichtung und verfügt über einen Austragfortsatz 22, an dessen distalem Ende eine Austragöffnung 23 vorgesehen ist. Gegenüber diesem äußeren Gehäuseabschnitt 20 ist ein innerer Gehäuseabschnitt 30 entlang der durch den Pfeil 2 symbolisierten Richtung relativbeweglich. Der innere Gehäuseabschnitt 30 wird im Wesentlichen durch einen Träger 32 sowie den Flüssigkeitsspeicher 12a eines Pumpspenders 12 gebildet, zu dem auch der Austragfortsatz 22 des äußeren Gehäuseabschnitts 20 gehört. Der Pumpspender 12 ist dafür ausgebildet, einen Austragvorgang durch die Austragöffnung 23 zu bewirken, wenn der Austragfortsatz 22 gegenüber dem Flüssigkeitsspeicher 12a in Richtung 2 verlagert wird. Hierzu weist er eine Pumpe 22a auf.

Die Austragvorrichtung verfügt weiterhin über ein elektronisches Zählermodul 50, welches am inneren Gehäuseabschnitt 30 vorgesehen ist, wobei im äußeren Gehäuseabschnitt 20 ein Sichtfenster 26 ausgespart ist, durch welches hindurch ein LC-Display oder ein bistabiles Display des Zählermoduls 50 abgelesen werden kann.

Zur Stromversorgung des Zählermoduls und von dessen elektronischen Komponenten weist die Austragvorrichtung eine Stromerzeugungseinheit 60 auf. Diese Stromerzeugungseinheit 60 umfasst als Hauptkomponenten einen elektromagnetischen Generator 70 sowie eine Antriebseinrichtung 80 für den Generator 70. Der Generator 70 verfügt über einen Stator 72 mit nicht dargestellten Spulenwicklungen, wobei dieser Stator 72 ortsfest zum Gehäuseabschnitt 30 angebracht ist. Er verfügt weiterhin über einen Rotor 74 mit einem innerhalb der Wicklungen des Stators 72 rotierenden Permanentmagneten. Durch Rotation des Rotors 74 gegenüber dem Stator 72 kann elektrische Energie erzeugt werden, die über nicht dargestellte Leitungen zum Zählermodul 50 geleitet wird.

Der Rotor 74 kann mittels eines zum Rotor drehfesten Zahnrades 76 in eine Drehbewegung gegenüber dem Stator 72 versetzt werden, um hierdurch die elektrische Energie zur Versorgung des Zählermoduls 50 zu erzeugen. Die hierfür vorgesehene Antriebseinrichtung 80 umfasst drei Teileinrichtungen, die bezogen auf die Richtung 2 gegeneinander relativbeweglich sind. Die erste dieser drei Teileinrichtungen ist ein sich in Richtung 2 erstreckender und zur Seite des Generators 70 offener Aufnahmeschacht 34, welcher Teil des inneren Gehäuseabschnitts 30 ist. Die zweite Teileinrichtung ist ein in diesen Schacht 34 eingeschobener und innerhalb des Schachtes 34 beweglicher hohl ausgebildeter Fortsatz 24, welcher ortsfest am äußeren Gehäuseabschnitt 20 vorgesehen ist. Die dritte Teileinrichtung ist ein Spannglied 82, welches ebenfalls zumindest zum überwiegenden Teil innerhalb des Schachtes 34 aufgenommen ist und welches gegenüber dem Fortsatz 24 begrenzt im Hinblick auf die Richtung 2 relativbeweglich ist. Dabei umfasst das Spannglied 82 einen Antriebsabschnitt 84, der derart an den Schacht 34 angepasst ist, dass er gegenüber diesem nicht drehbeweglich ist. Weiterhin umfasst das Spannglied 82 einen Auslöseabschnitt 86 mit einem radial nach außen weisenden Auslösestift 86a. Der Auslöseabschnitt 86 ist zusammen mit dem Antriebsabschnitt 84 in Richtung 2 beweglich. Er ist zusätzlich um die Hochachse 4 gegenüber dem Antriebsabschnitt 84 drehbeweglich. Zwischen dem oberseitigen Grund des Fortsatzes 24 und dem Spannglied 82 ist innerhalb des Fortsatzes 24 eine Druckfeder 88 vorgesehen. Der Antriebsabschnitt 84 des Spanngliedes weist eine kurze Zahnstange 84a auf, welche sich in der in Fig. 2a dargestellten Ausgangslage bereits in Eingriff mit dem Zahnrad 76 befindet.

Fig. 1 a verdeutlicht die begrenzte Relativbeweglichkeit des Spanngliedes 82 gegenüber dem Fortsatz 24. Der Fortsatz 24 weist eine Aussparung 24a auf, durch die hindurch der Auslösestift 86a ragt. Diese Aussparung 24a verfügt über zwei Schrägen 24b, 24c. Die obere Schräge 24b ist geeignet, den Auslösestift 86a zusammen mit dem Auslöseabschnitt 86 bezogen auf die Darstellung nach links zu verdrehen, wenn der Fortsatz 24 gegenüber dem Auslöseabschnitt 86 nach unten verlagert wird. Dies wird im Weiteren noch erläutert. Die untere Schräge 24c ist geeignet, den Auslösestift 86a zusammen mit dem Auslöseabschnitt 86 nach rechts zurückzudrücken, wenn der Auslöseabschnitt mittels der Feder 88 gegen diese untere Schräge 24c gedrückt wird.

Die Antriebseinrichtung 80 ist dafür vorgesehen, im Zuge einer manuellen Betätigung der Austragvorrichtung 10 zunächst zumindest einen Teil der hierbei in das System eingebrachten mechanischen Energie in der Feder 88 zu speichern. Diese gespeicherte Energie wird anschließend zum Teil in elektrische Energie für das Zählermodul 50 umgewandelt.

Im Einzelnen läuft dies wie folgt ab:
Ausgehend vom Zustand der Fig. 2a bis c werden die Gehäuseabschnitte 20, 30 in Richtung des Pfeils 2 relativ zueinander bewegt, wobei bezogen auf die Perspektive der Fig. 2a der äußere Gehäuseabschnitt 20 hinabgedrückt wird und/oder der innere Gehäuseabschnitt 30 hinaufgedrückt wird. Dies geschieht vorzugsweise dadurch, dass der Benutzer mindestens einen Finger auf der Oberseite des äußeren Gehäuseabschnitts 20 neben dem Austragfortsatz 22 und einen zweiten Finger oder den Daumen auf der Unterseite des Flüssigkeitsspeichers 12a platziert und dann eine aufeinander zu gerichtete Kraftbeaufschlagung und eine daraus resultierende Relativbewegung bewirkt.

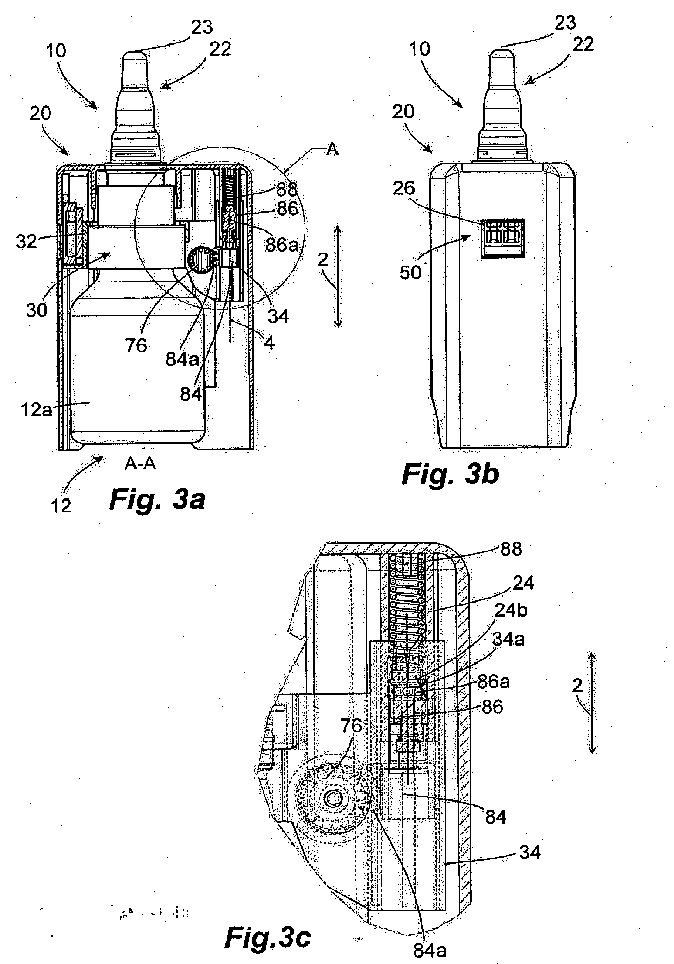

Die Fig. 3 bis 5 zeigen verschiedene Stadien dieser Betätigungsbewegung, wobei diese Betätigungsbewegung in der aus dem Stand der Technik bekannten üblichen Weise einen Austrag von Medium aus dem Mediumspeicher 12a durch die Austragöffnung 23 hindurch in die Umgebung bewirkt.

Ausgehend von Fig. 2 führt die genannte Relativbewegung der Gehäuseabschnitte 20, 30 zwangsläufig zu einer Verlagerung des Fortsatzes 24 gegenüber dem Schacht 34, da der Schacht 34 und der Fortsatz 24 jeweils ortsfest an jeweils einem Gehäuseabschnitt 20, 30 vorgesehen sind. Das Spannglied 82 bleibt zunächst in seiner in Fig. 2a dargestellten Relativlage relativ zum inneren Gehäuseabschnitt 30, da der Auslösestift 86a innerhalb einer horizontalen Ausnehmung 34a der Wandung des Schachtes 34 angeordnet ist und somit das Spannglied 82 bezogen auf die Richtung 2 am inneren Gehäuseabschnitt 30 festgelegt ist. Während dieser ersten Phase findet keine Erzeugung elektrischer Energie statt, da sich mit dem Spannglied 82 auch die Verzahnung 84a nicht gegenüber dem Generator 70 bewegt.

Durch die fortgesetzte Bewegung der Gehäuseabschnitte 20, 30 gegeneinander wird der Auslösestift 86a zunehmend nach links aus der Ausnehmung 34a hinausgedrückt, wobei dies mittels der in Fig. 3c eingezeichneten Schräge 24b am Fortsatz 24 erfolgt, welche durch die Relativbewegung der Gehäuseabschnitte 20, 30 gemeinsam mit dem äußeren Gehäuseabschnitt 20 verlagert wird. Ermöglicht wird diese Bewegung des Auslösestiftes 86a durch die Drehbeweglichkeit des Auslöseabschnitts 86 gegenüber dem Antriebsabschnitt 84 des Spanngliedes 82.

Fig. 3a und 3c, insbesondere Fig. 3c, zeigen die veränderte Lage des Auslösestiftes 86a gegenüber der Ausnehmung 34a bei fortgesetzter Betätigungsbewegung.

In dieser ersten Phase der Betätigung, während der Auslösestift 86a sich in der Ausnehmung 34a befindet, bewegen sich der innere Gehäuseabschnitt 30 und das Spannglied 82 zwangsläufig gemeinsam, so dass es zu einem Spannen der Feder 88 kommt, welche mit fortschreitender Relativbewegung der Gehäuseabschnitte 20, 30 komprimiert wird.

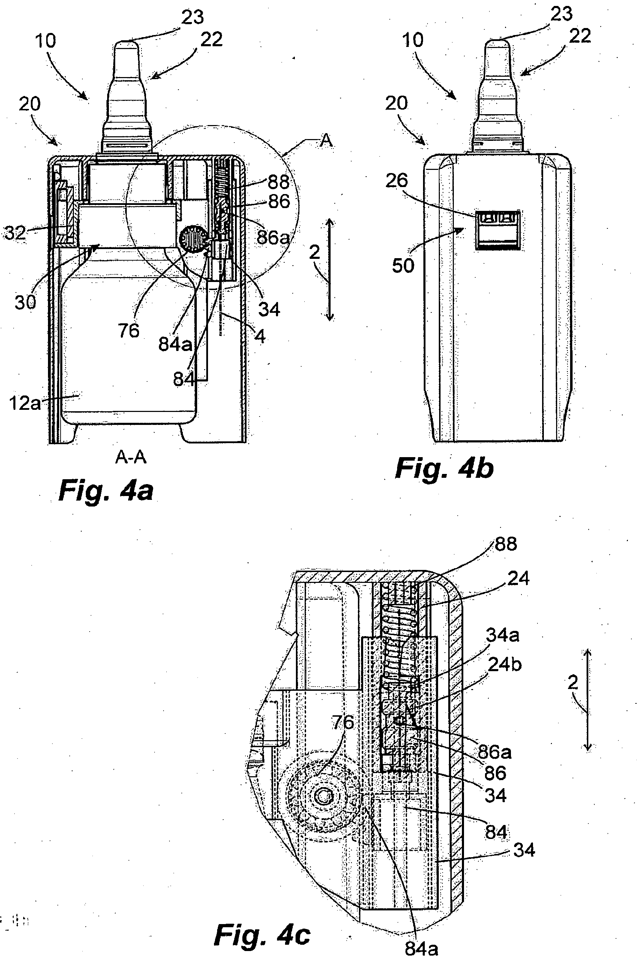

Sobald die Gehäuseabschnitte 20, 30 so weit verlagert sind, dass der Auslösestift 86a mittels der Schräge 24b vollständig nach links aus der Ausnehmung 34a herausgedrückt wurde, folgt eine zweite Phase der Betätigung, die in Figur 4 dargestellt ist und die die Hauptphase der Stromerzeugung bildet. In dieser Hauptphase der Stromerzeugung gibt die unter Spannung stehende Feder 88 die in ihr gespeicherte Federenergie frei und drückt dadurch das Spannglied 82 gegenüber dem äußeren Gehäuseabschnitt 20 und insbesondere auch gegenüber dem inneren Gehäuseabschnitt 30 nach unten. Durch die Relativbewegung des Spanngliedes 82 gegenüber dem inneren Gehäuseabschnitt 30 bewegt sich in dieser Phase auch die Zahnstange 84a gegenüber dem Generator 70 und treibt somit das Zahnrad 76 an. Da dies in erster Linie durch die vorgespannte Feder 88 bewirkt wird, wird hier eine sehr hohe Geschwindigkeit erreicht. Das Zahnrad und somit der Rotor des Generators 70 werden bis auf etwa 5000 U/min beschleunigt. Diese Geschwindigkeit ist zur Erzeugung elektrischer Energie sehr gut geeignet.

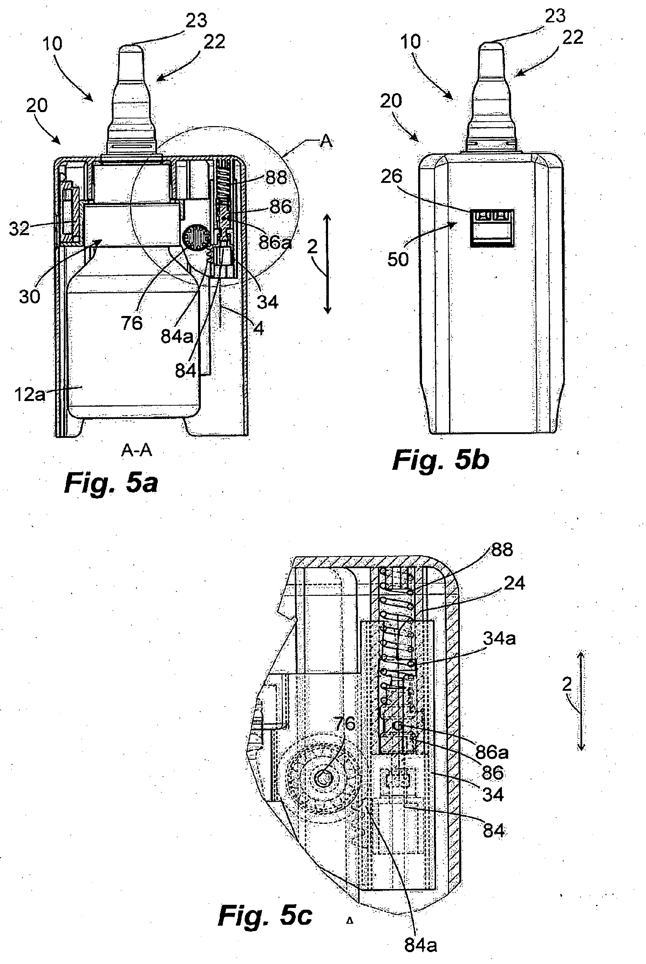

Durch die fortgesetzte Betätigungsbewegung der Gehäuseabschnitte 20, 30 gegeneinander und die Relativbewegung des Spanngliedes 82 gegenüber den Gehäuseabschnitten 20, 30 gelangt die Zahnstange 84a in der in Fig. 5 dargestellten Weise gegen Ende ihrer Bewegung außer Eingriff vom Zahnrad 76. Dies gestattet es, dass das Zahnrad 76 zu jenem Zeitpunkt, zu dem das Spannglied 82 seine Endlage der Fig. 5 erreicht, nicht schlagartig seine Drehbewegung und damit die Drehbewegung des Rotors einstellt, sondern stattdessen nachlaufen kann. Somit wird auch nach Stillstand des Spanngliedes 82 relativ zum inneren Gehäuseabschnitt 30 weiterhin elektrische Energie in einer Nachlaufphase erzeugt. Auf diese Art und Weise lassen sich etwa 10% bis 25% mehr elektrische Energie erzeugen, als wenn die Zahnstange nicht außer Eingriff mit dem Zahnrad gelangt und somit bei Erreichen der Endlage des Spanngliedes 82 gegenüber dem inneren Gehäuseabschnitt 30 das Zahnrad und damit den Rotor abrupt bremst.

Die erzeugte Energie wird verwendet, um ein elektronisches Zählregister im Zählermodul 50 um eins zu erhöhen und den Wert zumindest kurzzeitig auf dem LC-Display des Zählermoduls 50 anzuzeigen. Bei Verwendung eines bistabiles Displays bleibt diese Anzeige bis zur nächsten Änderung erhalten.

Nach Wegfall der externen Kraftbeaufschlagung werden die Gehäuseabschnitte 20, 30 unter Einwirkung einer internen und nicht dargestellten Rückstellfeder innerhalb des Pumpspenders 12 wieder in Ihre Ausgangslage der Fig. 2 zurückgedrückt. Der Auslösestift 86a wird aufgrund der Federkraft der Feder 88 und aufgrund der Schräge 24c zurück in die Ausnehmung 34a der Wandung des Schachtes 34 eingefahren.
Bei der dargestellten Ausführungsform erfolgt das Auslösen des Spanngliedes 82 und somit der Beginn der Erzeugung elektrischer Energie noch bevor die Gehäuseabschnitte 20, 30 ihre Relativendlage erreicht haben. Bei alternativen Ausgestaltungen kann jedoch vorgesehen sein, dass erst gegen Ende, also im Bereich der letzten 10% des Hubweges der Gehäuseabschnitte 20, 30 gegeneinander, die Auslösung erfolgt.

## Patentansprüche

1. Austragvorrichtung (10) für flüssige, pastöse oder pulverförmige Medien mit
- einem Gehäuse mit einem ersten Gehäuseabschnitt (30) und einem zweiten Gehäuseabschnitt (20), die gegeneinander zum Zwecke der Betätigung hubbeweglich sind,
- einer Austragöffnung (23) zum Austrag des Mediums und
- einem Reservoir (12a) zur Speicherung des Mediums vor dessen Austrag,
wobei
- durch eine manuelle Hubbewegung der Gehäuseabschnitte (20, 30) gegeneinander eine Förderung von Medium vom Reservoir (12a) zur Austragöffnung (23) bewirkt werden kann,
- die Austragvorrichtung (10) einen elektrischen Verbraucher (50) aufweist,
- die Austragvorrichtung einen elektromagnetischen Generator (70) zur Umwandlung der bei der Betätigung eingebrachten mechanischen Energie in elektrische Energie zur Versorgung des elektrischen Verbrauchers (50) aufweist, und
- der elektromagnetische Generator (70) eine Komponente (74), die einen Magneten umfasst, und eine Komponenten (72), die einen mit dem elektrischen Verbraucher (50) verbundenen Leiter umfasst, aufweist, wobei eine der Komponenten (72) ortsfest zu einem der Gehäuseabschnitte (30) und eine der Komponenten (74) gegenüber der ortsfesten Komponenten (72) beweglich vorgesehen ist,
**gekennzeichnet durch**
einen Federenergiespeicher mit einem gegenüber einem zweiten Gehäuseabschnitte beweglichen Spannglied (82) und einer zwischen dem zweiten Gehäuseabschnitt (20) und dem Spannglied (82) wirkenden Feder (88), wobei das Spannglied
- dafür ausgebildet ist, während der Hubbewegung mit dem ersten Gehäuseabschnitt (30) gekoppelt zu sein, so dass ein Spannen der Feder (88) bewirkt wird,
- im gespannten Zustand der Feder (88) vom ersten Gehäuseabschnitt (20) entkoppelbar ist, so dass ein Entspannen der Feder (88) ermöglicht wird, und
- während des Entspannens der Feder (88) mit der beweglichen Komponente (74) des Generators zumindest phasenweise wirkgekoppelt ist, so dass die Bewegung des Spanngliedes (82) eine Bewegung der beweglichen Komponente bewirkt (74).

2. Austragvorrichtung (10) nach Anspruch 1,
**dadurch gekennzeichnet, dass**
die bewegliche Komponente (74) des Generators (70) gegenüber der ortsfesten Komponente (70) drehbeweglich gelagert ist.

3. Austragvorrichtung (10) nach Anspruch 1 oder 2,
**dadurch gekennzeichnet, dass**
das Spannglied (82) und der erste und zweite Gehäuseabschnitt (20, 30) derart ausgebildet und aufeinander abgestimmt sind, dass das Spannglied (82) in einer definierten Entkoppelungsstellung der Hubbewegung automatisch vom ersten Gehäuseabschnitt (30) entkoppelt wird.

4. Austragvorrichtung (10) nach Anspruch 3,
**dadurch gekennzeichnet, dass**
die Entkopplungsstellung und die Feder (88) derart aufeinander abgestimmt sind, dass während des Entspannens der Feder die bewegte Komponente (74) des Generators (70) mindestens eine Maximalgeschwindigkeit von 800 mm/sec oder mindestens eine Maximaldrehgeschwindigkeit von 30 Umdrehungen/sec erreicht.

5. Austragvorrichtung (10) nach einem der vorstehenden Ansprüche,
**dadurch gekennzeichnet, dass**
das Spannglied (82) während des Entspannens der Feder (88) zunächst mit der beweglichen Komponente (74) des Generators (70) wirkgekoppelt wird, wobei diese Wirkkopplung derart ausgebildet ist, dass sie noch vor Erreichen des Stillstandes des Spanngliedes (82) endet.

6. Austragvorrichtung (10) nach einem der vorstehenden Ansprüche,
**dadurch gekennzeichnet, dass**
die Wirkkoppelung zwischen dem Spannglied (82) und der beweglichen Komponenten (74) über Verzahnungen (84a) an der beweglichen Komponente (74) und am Spannglied (82) erfolgt.

7. Austragvorrichtung nach einem der vorstehenden Ansprüche,
**dadurch gekennzeichnet, dass**
die Gehäuseabschnitte einer Betätigungsrichtung gegeneinander beweglich sind, die mit einer durch eine Austragrichtung des Mediums definierte Haupterstreckungsrichtung der Austragvorrichtung einen Winkel zwischen 70° und 110° einschließt.

8. Austragvorrichtung nach einem der vorstehenden Ansprüche,
**dadurch gekennzeichnet, dass**
zur Förderung des Mediums zur Austragöffnung (23) eine Förderpumpe (22a) oder zum Auslass des Mediums zur Austragvorrichtung ein Auslassventil vorgesehen ist.

9. Austragvorrichtung nach Anspruch 8,
**dadurch gekennzeichnet, dass**
die Förderpumpe (22a) oder das Auslassventil derart ausgebildet ist, dass ein Austrag des Mediums im Zuge einer Hubbewegung jenseits der Entkoppelungsstellung erfolgt.

## Claims

1. A discharge device (10) for liquid, pasty or pulverulent media, having
- a housing with a first housing portion (30) and a second housing portion (20), which are displaceable in relation to one another for actuating purposes,
- a discharge opening (23) for discharging the medium, and
- a reservoir (12a) for storing the medium prior to being discharged,
wherein
- a manual displacement movement of the housing portions (20, 30) in relation to one another can cause medium to be delivered from the reservoir (12a) to the discharge opening (23),
- the discharge device (10) has an electric load (50),
- the discharge device has an electromagnetic generator (70), by means of which the mechanical energy introduced upon actuation is converted into electrical energy for supplying the electric load (50), and
- the electromagnetic generator (70) has a component (74) which comprises a magnet and a component (72) which comprises a conductor connected to the electric load (50), wherein one of the components (72) is provided at a fixed location in relation to one of the housing portions (30) and one of the components (74) is provided such that it can be moved in relation to the fixed-location component (72),
**characterized by**
a spring energy store with a stressing member (82), which can be moved in relation to a second housing portion, and a spring (88), which acts between the second housing portion (20) and the stressing member (82), wherein the stressing member
- is designed to be coupled to the first housing portion (30) during the displacement movement, and therefore the spring (88) is subjected to stressing,
- in the stressed state of the spring (88), can be uncoupled from the first housing portion (20), and this therefore allows the spring (88) to be relieved of stressing, and
- while the spring (88) is being relieved of stressing, is operatively coupled to the movable component (74) of the generator at least in certain phases, and therefore the movement of the stressing member (82) causes the movable component (74) to move.

2. The discharge device (10) according to claim 1, **characterized in that** the movable component (74) of the generator (70) is mounted such that it can be rotated in relation to the fixed-location component (70).

3. The discharge device (10) according to claim 1 or 2, **characterized in that** the stressing member (82) and the first and second housing portions (20, 30) are designed, and co-ordinated with one another, such that, in a defined uncoupling position of the displacement movement, the stressing member (82) is uncoupled automatically from the first housing portion (30).

4. The discharge device (10) according to claim 3, **characterized in that** the uncoupling position and the spring (88) are co-ordinated with one another such that, as the spring is being relieved of stressing, the moving component (74) of the generator (70) reaches at least a maximum speed of 800 mm/sec or at least a maximum rotational speed of 30 revolutions/sec.

5. The discharge device (10) according to any one of the preceding claims, **characterized in that** as the spring (88) is being relieved of stressing, the stressing member (82) is operatively coupled in the first instance to the movable component (74) of the generator (70), wherein said operative coupling is such that it terminates even prior to the stressing member (82) reaching a standstill.

6. The discharge device (10) according to any one of the preceding claims, **characterized in that** the operative coupling between the stressing member (82) and the movable component (74) takes place via toothing formations (84a) on the movable component (74) and on the stressing member (82).

7. The discharge device according to any one of the preceding claims, **characterized in that** the housing portions can be moved in relation to one another in an actuating direction which encloses an angle between 70° and 110° with a main direction of extent of the discharge device, this latter direction being defined by a discharging direction of the medium.

8. The discharge device according to any one of the preceding claims, **characterized in that** a delivery pump (22a) is provided for delivering the medium to the discharge opening (23) or an outlet valve is provided for letting the medium out of the discharge device.

9. The discharge device according to claim 8, **characterized in that** the delivery pump (22a) or the outlet valve is designed such that the medium is discharged during the course of a displacement movement beyond the uncoupling position.

## Revendications

1. Dispositif de distribution (10) pour des milieux fluides, pâteux ou pulvérulents, comprenant
- un boîtier avec une première portion de boîtier (30) et une deuxième portion de boîtier (20) qui peuvent être animées d'un mouvement de course l'une par rapport à l'autre en vue de l'actionnement,
- une ouverture de distribution (23) pour distribuer le milieu et
- un réservoir (12a) pour stocker le milieu avant sa distribution,
dans lequel
- un refoulement de milieu depuis le réservoir (12a) jusqu'à l'ouverture de distribution (23) peut être effectué par un mouvement de course manuel des portions de boîtier (20, 30) l'une par rapport à l'autre,
- le dispositif de distribution (10) présente un consommateur électrique (50),
- le dispositif de distribution présente un générateur électromagnétique (70) pour convertir l'énergie mécanique introduite lors de l'actionnement en énergie électrique pour l'alimentation du consommateur électrique (50), et
- le générateur électromagnétique (70) présente un composant (74) qui comprend un aimant, et un composant (72) qui comprend un conducteur connecté au consommateur électrique (50), l'un des composants (72) étant prévu fixement par rapport à l'une des portions de boîtier (30) et l'un des composants (74) étant prévu de manière déplaçable par rapport au composant fixe (72),
**caractérisé par**
un accumulateur d'énergie à ressort avec un organe de serrage (82) déplaçable par rapport à une deuxième portion de boîtier et avec un ressort (88) agissant entre la deuxième portion de boîtier (20) et l'organe de serrage (82), l'organe de serrage
- étant réalisé de manière à être accouplé pendant le mouvement de course avec la première portion de boîtier (30), de sorte qu'un serrage du ressort (88) soit réalisé,
- pouvant être désaccouplé dans l'état serré du ressort (88) de la première portion de boîtier (20), de sorte qu'une détente du ressort (88) soit possible, et
- étant accouplé fonctionnellement au moins par phase pendant la détente du ressort (88) avec le composant déplaçable (74) du générateur de sorte que le mouvement de l'organe de serrage (82) provoque un mouvement du composant déplaçable (74).

2. Dispositif de distribution (10) selon la revendication 1,
**caractérisé en ce que**
le composant déplaçable (74) du générateur (70) est supporté de manière déplaçable en rotation par rapport au composant fixe (70).

3. Dispositif de distribution (10) selon la revendication 1 ou 2,
**caractérisé en ce que**
l'organe de serrage (82) et la première et la deuxième portion de boîtier (20, 30) sont réalisés et adaptés les uns aux autres de telle sorte que l'organe de serrage (82), dans une position de désaccouplement définie du mouvement de course, soit automatiquement désaccouplé de la première portion de boîtier (30).

4. Dispositif de distribution (10) selon la revendication 3,
**caractérisé en ce que**
la position de désaccouplement et le ressort (88) sont adaptés l'un à l'autre de telle sorte que pendant la détente du ressort, le composant déplacé (74) du générateur (70) atteigne au moins une vitesse maximale de 800 mm/s ou au moins une vitesse de rotation maximale de 30 rotations par seconde.

5. Dispositif de distribution (10) selon l'une quelconque des revendications précédentes,
**caractérisé en ce que**
l'organe de serrage (82), pendant la détente du ressort (88), est accouplé fonctionnellement d'abord au composant déplaçable (74) du générateur (70), cet accouplement fonctionnel étant réalisé de telle sorte qu'il se termine encore avant d'atteindre l'arrêt de l'organe de serrage (82).

6. Dispositif de distribution (10) selon l'une quelconque des revendications précédentes,
**caractérisé en ce que**
l'accouplement fonctionnel entre l'organe de serrage (82) et le composant déplaçable (74) s'effectue par le biais de dentures (84a) sur le composant déplaçable (74) et sur l'organe de serrage (82).

7. Dispositif de distribution selon l'une quelconque des revendications précédentes,
**caractérisé en ce que**
les portions de boîtier sont déplaçables l'une par rapport à l'autre dans une direction d'actionnement, qui forme avec une direction d'étendue principale définie par une direction de distribution du milieu du dispositif de distribution un angle compris entre 70° et 110°.

8. Dispositif de distribution selon l'une quelconque des revendications précédentes,
**caractérisé en ce que**
l'on prévoit, pour le refoulement du milieu jusqu'à l'ouverture de distribution (23), une pompe de refoulement (22a) ou, pour la sortie du milieu jusqu'au dispositif de distribution, une soupape de sortie.

9. Dispositif de distribution selon la revendication 8,
**caractérisé en ce que**
la pompe de refoulement (22a) ou la soupape de sortie sont réalisées de telle sorte qu'une distribution du milieu se produise au cours d'un mouvement de course au-delà de la position de désaccouplement.
